(19) Europäisches Patentamt
European Patent Office
Office européen des brevets

(11) Veröffentlichungsnummer: **0 184 712**
**A2**

(12) # EUROPÄISCHE PATENTANMELDUNG

(21) Anmeldenummer: **85114959.1**

(22) Anmeldetag: **26.11.85**

(51) Int. Cl.[4]: **C 09 B 19/02,** C 07 D 498/02, C 07 D 498/22, C 07 D 513/22

(30) Priorität: **08.12.84 DE 3444888**

(43) Veröffentlichungstag der Anmeldung: **18.06.86**
**Patentblatt 86/25**

(84) Benannte Vertragsstaaten: **CH DE FR GB LI**

(71) Anmelder: **BAYER AG, Konzernverwaltung RP**
**Patentabteilung, D-5090 Leverkusen 1 Bayerwerk (DE)**

(72) Erfinder: **Franke, Günter, Dr.,**
**Landrat-Trimborn-Strasse 60, D-5653 Leichlingen 1 (DE)**

(54) **Herstellung von Dioxazinverbindungen.**

(57) Verfahren zur Herstellung von Dioxazinverbindungen der Formel

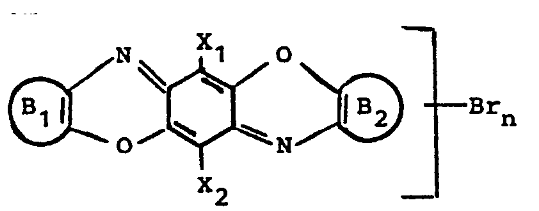

(I)

in der

$B_1$, $B_2$ ein Ringsystem mit 1, 2, 3 oder 4 carbo- und/oder heterocyclischen Ringen, die substituiert sein können und

$X_1$, $X_2$ Wasserstoff, Halogen, -R', -OR', -NHR',

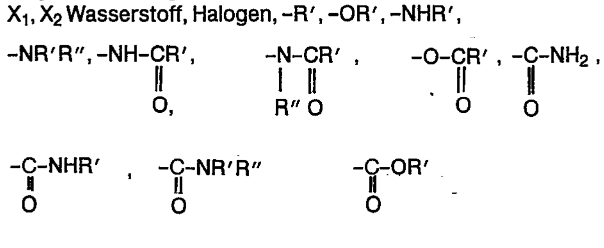

bezeichnen, wobei

R', R'' für Alkyl, Cycloalkyl, Aryl, Aralkyl stehen und

n 2, 3, 4, 5, 6, 7, 8, 9, 10, 11 bedeutet, wobei die n Bromatome unabhängig voneinander sowohl H-Atome der Ringsysteme $B_1$, $B_2$ oder ihrer Substituenten als auch H-Atome von $X_1$, $X_2$ sowie $X_1$, $X_2$ selbst ersetzen können, dadurch gekennzeichnet, daß man Verbindungen der Formel,

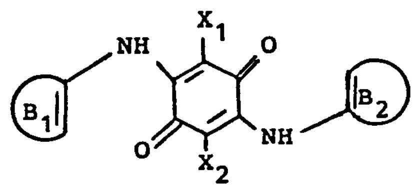

(II)

vorzugsweise in Gegenwart von Verdünnungsmitteln, mit Brom umsetzt.

EP 0 184 712 A2

BAYER AKTIENGESELLSCHAFT 5090 Leverkusen, Bayerwerk
Konzernverwaltung RP
Patentabteilung PG/m-c

## Herstellung von Dioxazinverbindungen

Die Erfindung betrifft ein Verfahren zur Herstellung von Dioxazinverbindungen der Formel

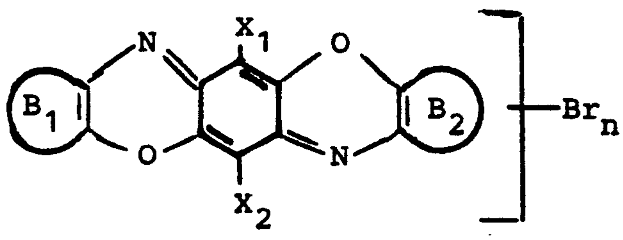

(I)

in der

$B_1$, $B_2$ ein Ringsystem mit 1, 2, 3 oder 4 carbo- und/oder heterocyclischen Ringen, die substituiert sein können und

$X_1$, $X_2$ Wasserstoff, Halogen, -R', -OR', -NHR', -NR'R", $-NH-\underset{\overset{\|}{O}}{C}R'$, $-\underset{\overset{|}{R''}}{N}-\underset{\overset{\|}{O}}{C}R'$, $-O-\underset{\overset{\|}{O}}{C}R'$, $-\underset{\overset{\|}{O}}{C}-NH_2$, $-\underset{\overset{\|}{O}}{C}-NHR'$, $-\underset{\overset{\|}{O}}{C}-NR'R''$, $-\underset{\overset{\|}{O}}{C}-OR'$

bezeichnen, wobei

R', R" für Alkyl, Cycloalkyl, Aryl, Aralkyl stehen und n 2, 3, 4, 5, 6, 7, 8, 9, 10, 11 bedeutet, wobei die n Bromatome unabhängig voneinander sowohl H-Atome der Ringsysteme $B_1$, $B_2$ oder ihrer Substituenten als auch H-Atome von $X_1$, $X_2$ sowie $X_1$, $X_2$ selbst ersetzen können, das dadurch gekennzeichnet ist, daß man Verbindungen der Formel

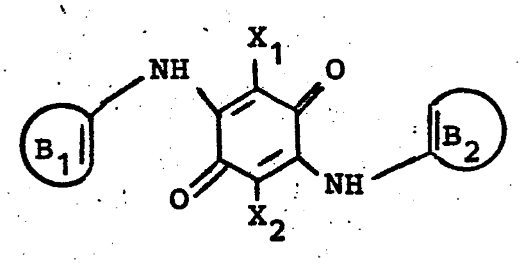

(II)

vorzugsweise in Gegenwart von Verdünnungsmitteln, mit Brom umsetzt.

In besonders gelagerten Fällen entstehen beim erfindungsgemäßen Verfahren die Oxidationsprodukte der Verbindungen der Formel (I). Für den Fall, daß (II) Substituenten $X_1$, $X_2$ enthält, die bei der erfindungsgemäßen Reaktion zumindest teilweise gegen Brom ausgetauscht werden, führt die Umsetzung von (II), z.B. für den Fall, daß $X_1 = X_2$ zu Verbindungen der Formel

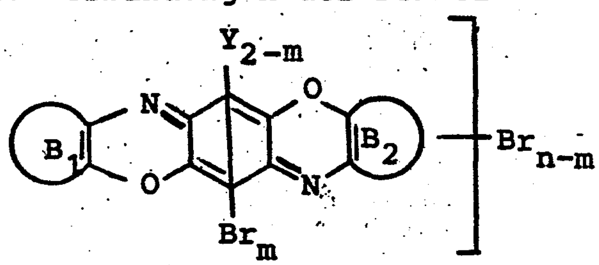

(III)

wobei Y einen der für $X_1$, $X_2$ genannten Substituenten, der beim erfindungsgemäßen Verfahren gegen Brom ausgetauscht werden kann wie Chlor oder Wasserstoff bezeichnet und n die oben angegebene Bedeutung hat, wobei für m $0 < m \leq 2$ gilt.

Als Substituenten der Ringsysteme $B_1$, $B_2$ kommen vorzugsweise Halogen, -R', -OR', -NHR', -NR'R", $-NH-\underset{\overset{\|}{O}}{C}-R'$,

$-\underset{R''}{N}-\underset{\overset{\|}{O}}{C}R'$, $-O-\underset{\overset{\|}{O}}{C}R'$, $-\underset{\overset{\|}{O}}{C}NH_2$, $-\underset{\overset{\|}{O}}{C}-NHR'$, $-\underset{\overset{\|}{O}}{C}-NR'R''$, $-\underset{\overset{\|}{O}}{C}-OR'$, -OH,

-SR', -CN, -SCN, $-NO_2$, $-NH_2$, -R'-O-R", $R'\underset{\overset{\|}{O}}{C}-$, und $R'SO_2-$

in Betracht, wobei R' und R" die oben angegebenen Bedeutungen haben.

Die Ringsysteme $B_1$, $B_2$ bestehen vorzugsweise aus 2, 3 oder 4 kondensierten aromatischen 5- und 6-gliedrigen Ringen, die Heteroatome wie N, O, S enthalten können. In den folgenden Beispielen geben die mit x markierten Stellen die beim Ringschluß entstehende Bindung zum Sauerstoff an; die mit y markierten Stellen geben die Bindung zum N an:

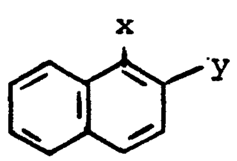
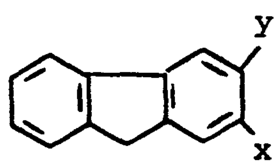
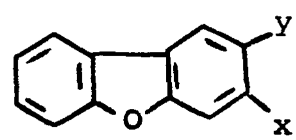

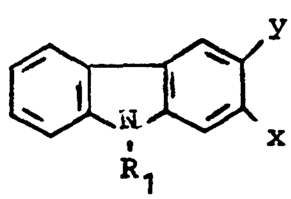
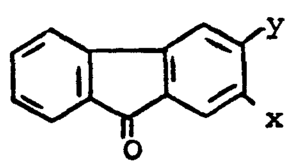
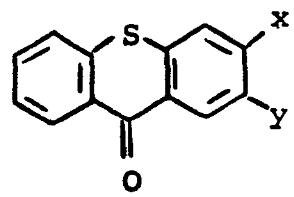

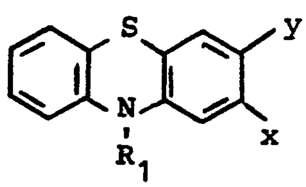 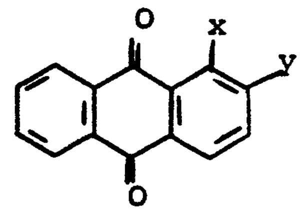 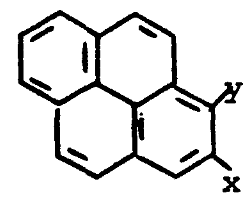

worin

$R_1$ bezeichnet Wasserstoff, $R_2$-, $-R_2-O-R_3-$, $R_2\underset{O}{\underset{\|}{C}}-$, $R_2-SO_2-$,

wobei $R_2$, $R_3$, Alkyl, Cycloalkyl, Aryl und Aralkyl bezeichnen.

Im Zusammenhang mit Formel (I) stehen Halogen bevorzugt für Chlor und Brom, Alkyl bevorzugt für $C_1$-$C_4$-Alkyl, Cycloalkyl bevorzugt für Cyclopentyl und Cyclohexyl, Aryl bevorzugt für Phenyl und p-Tolyl sowie Aralkyl bevorzugt für Benzyl und Phenylethyl.

Das erfindungsgemäße Verfahren führt man vorteilhaft in hochsiedenden, unter den Reaktionsbedingungen inerten Lösungsmitteln z.B. Nitrobenzol oder chlorierte Kohlenwasserstoffe wie Pentachlorpropan, o-Dichlorbenzol oder Trichlorbenzol bei 10-270°C, vorzugsweise bei 130°-250°C, besonders 170°-220°C, gegebenenfalls unter Druck und gegebenenfalls in einer Inertgasatmosphäre durch. Man kann die Reaktion auch direkt in Brom durchführen.

Die Herstellung der Vorprodukte (II) erfolgt beispielsweise dadurch, daß man gemäß untenstehendem Formelschema Aminoverbindungen der Formeln (IVa) und (IVb) mit Chinonen der Formel (V) kondensiert:

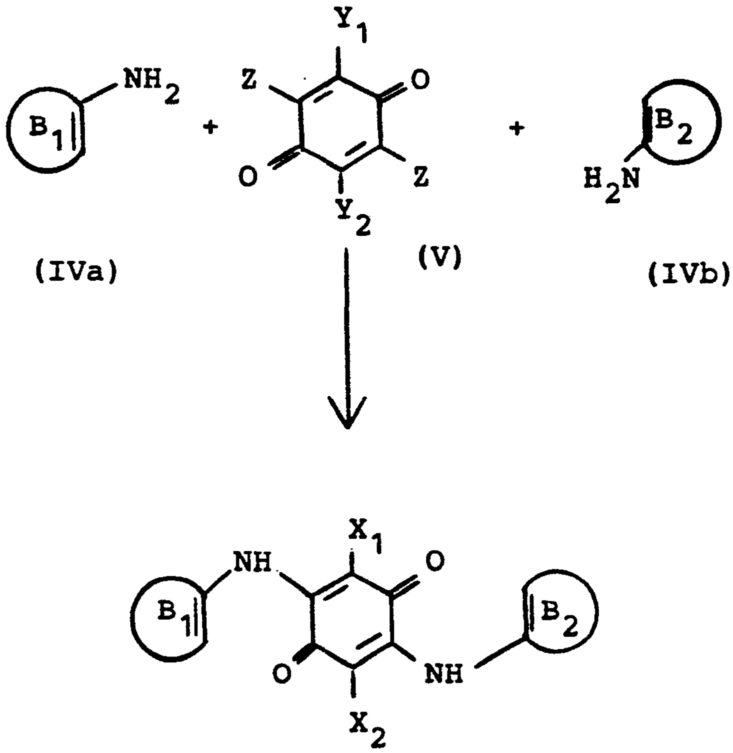

(IVa) (V) (IVb)

(II)

Z bezeichnet vorzugsweise Halogen, insbesondere Chlor, Hydroxy, Alkoxy und Aryloxy, insbesondere Phenoxy.

Als "Mittelkomponenten" der Formel (V) kommen z.B. Verbindungen der Formeln (VI) und (VII) in Frage

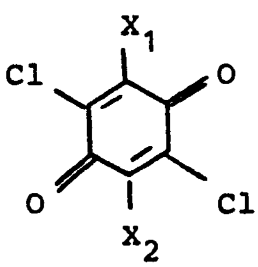

(VI)

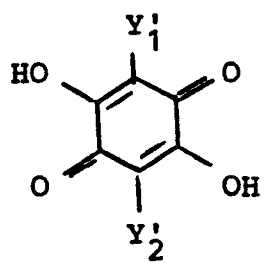

(VII)

worin $Y_1'$ und $Y_2'$ das gleiche wie $X_1$ und $X_2$, jedoch kein Halogen bedeuten.

Es ist vorteilhaft, wo immer möglich, die Gewinnung von (II) und die nachfolgende Umsetzung zum Pigment (I) ohne Zwischenisolierung von (II) durchzuführen.

Setzt man bei der erfindungsgemäßen Reaktion von den Vorprodukten der Formel (II) die der Formel (VIII) ein, erhält man gemäß dem nachfolgenden Schema in einigen Fällen statt der entsprechenden Dioxazine ihre durch Weiteroxidation entstehenden Folgeprodukte der Formel (IX):

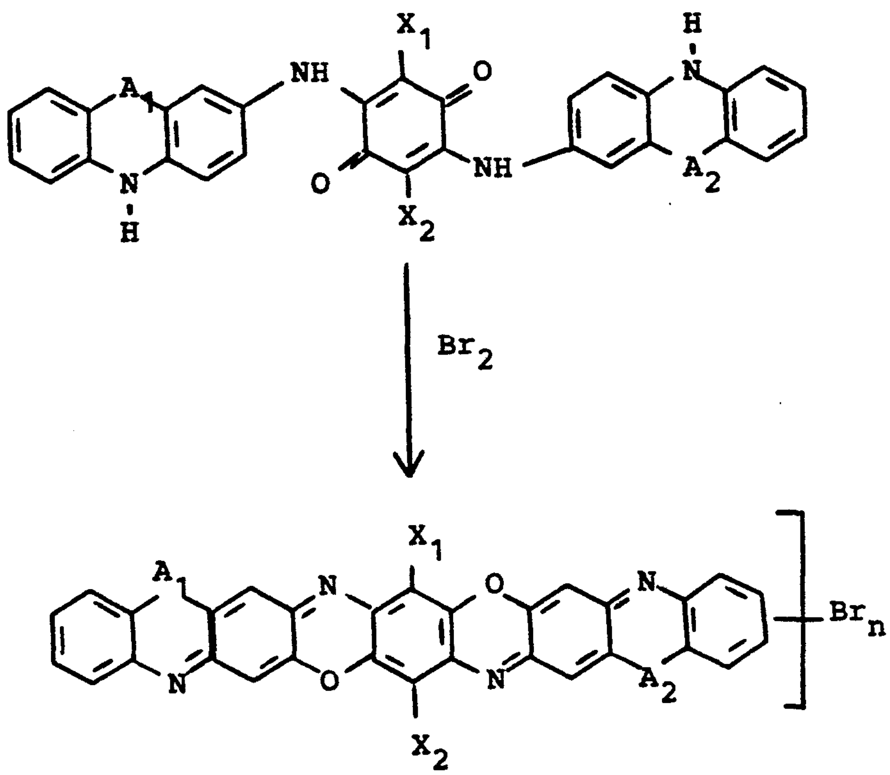


(VIII)

(IX)

In diesem Schema bedeuten $A_1$ und $A_2$ unabhängig voneinander eine direkte Bindung oder ein Heteroatom wie O, S oder $NR_1$. Die Verbindungen (VIII) und (IX) können ferner ankondensierte Benzolringe besitzen. Auch die Verbindungen der Formel (IX) stellen wertvolle Pigmente dar.

Von den Vorprodukten der Formel (II) sind die der Formel (X) als für das erfindungsgemäße Verfahren besonders geeignet zu nennen

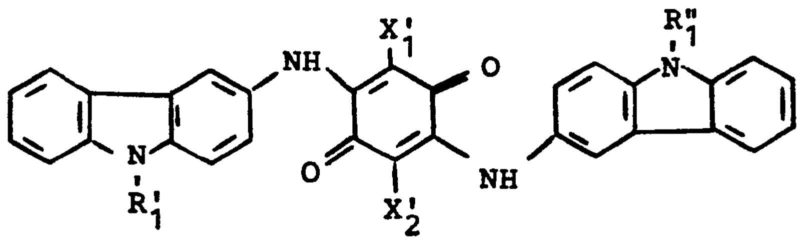

(X)

worin

| | |
|---|---|
| $X_1'$ und $X_2'$ | dasgleiche bedeuten wie $X_1$ und $X_2$ und |
| $R_1'$ und $R_1''$ | unabhängig voneinander die Bedeutung von $R_1$ haben. |

In den bevorzugten Verbindungen (X) sind $X_1'$ und $X_2'$ bzw. $R_1'$ und $R_1''$ gleich.

Von den für das erfindungsgemäße Verfahren bevorzugten Verbindungen (X) sind besonders diejenigen zu nennen, für die gilt:

$X_1'$ und $X_2'$ = Wasserstoff oder Chlor
$R_1'$ und $R_1''$ = $C_1$-$C_4$-Alkyl, vorzugsweise Ethyl.

Führt man die erfindungsgemäße Reaktion mit den hierfür besonders geeigneten Verbindungen der Formel (X) durch, sind bezüglich der Brommenge jene Verfahrensweisen hervorzuheben, die zu Verbindungen der Formel (XI) führen

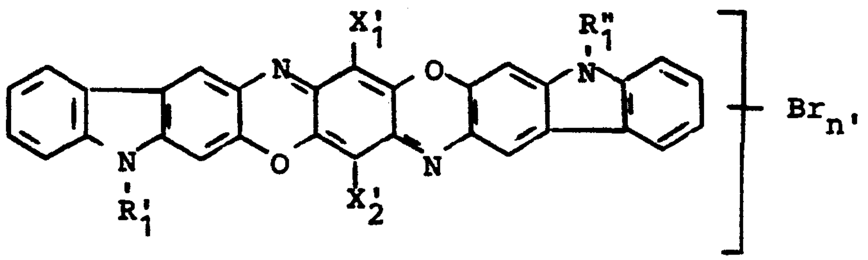

(XI)

worin für n' = 4, 5, 6, 7, 8 gilt und $R_1'$ und $R_1''$ bzw. $X_1'$ und $X_2'$ die zuvor genannten Bedeutungen haben.

Weil für den Fall $X_1'$ = $X_2'$ = Wasserstoff oder Chlor bei der erfindungsgemäßen Reaktion immer ein Teil von $X_1'$ und $X_2'$ gegen Brom ausgetauscht wird, lassen sich die Verbindungen der Formel (XI) auch in der Formel (XII) darstellen,

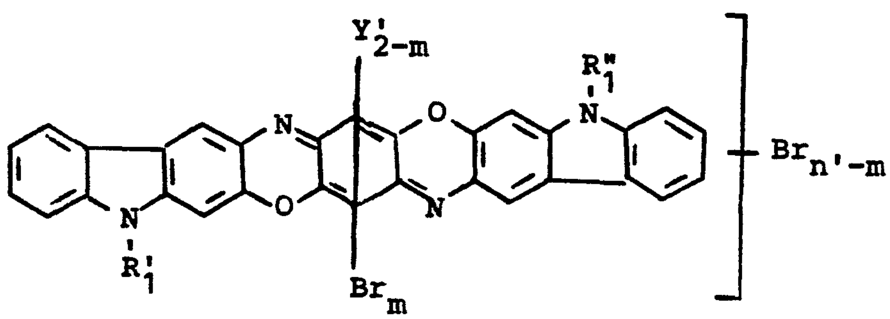

(XII)

worin gilt

Y' = Wasserstoff oder Chlor

$0 < m \leq 2$

und worin $R_1'$, $R_1''$ und n' die zuvor genannten Bedeutungen haben.

Die nach dem erfindungsgemäßen Verfahren gewonnenen Verbindungen der Formel (I) eignen sich, vorzugsweise nach üblicher Trocken- oder Naßkonditionierung, aufgrund ihrer guten Pigmenteigenschaften für die verschiedensten Pigmentapplikationen. So können sie zur Herstellung von sehr echt pigmentierten Systemen, wie Mischungen mit anderen Stoffen, Zubereitungen, Anstrichmitteln, Druckfarben, gefärbtem Papier und gefärbten makromolekularen Stoffen verwendet werden. Unter Mischung mit anderen Stoffen können z.B. solche mit anorganischen Weißpigmenten wie Titandioxid (Rutil) verstanden werden. Zubereitungen sind z.B. Flushpasten mit organischen Flüssigkeiten und gegebenenfalls Konservierungsmitteln. Die Bezeichnung Anstrichmittel steht z.B. für physikalisch oder oxidativ trocknende Lacke, Einbrennlacke, Reaktionslacke, Zweikomponentenlacke, Dispersionsfarben für wetterfeste Überzüge und Leimfarben. Unter Druckfarben sind solche für den Papier-, Textil- und Blechdruck zu verstehen. Insbesondere eignen sich erfindungsgemäß gewonnene Pigmente für Anstrichmittel und zum Einsatz in Automobillacken, namentlich für Metalliclackierungen. Sie sind ausgezeichnet wasser- und lösungsmittelecht, überlackier- und überspritzecht, sublimierecht und insbesondere ausgezeichnet licht- und wetterecht. Bei hoher Brillanz ist ihre Nuance deutlich röter als die der bromfreien Verbindungen.

## Beispiele

### Beispiel 1

a) 17,5 g 2,5-Dihydroxy-p-benzochinon und 56,0 g 3-Amino-9-ethylcarbazol werden in einer Mischung aus 530 ml Eisessig und 25 ml 10 proz. Schwefelsäure 2 Stunden unter Rückfluß erhitzt. Nach Erkalten wird der Ansatz abgesaugt, mit 400 ml Eisessig, dann mit 2 l heißem Wasser gewaschen und bei 100°C getrocknet. Man erhält 60,0 g der Verbindung der Formel

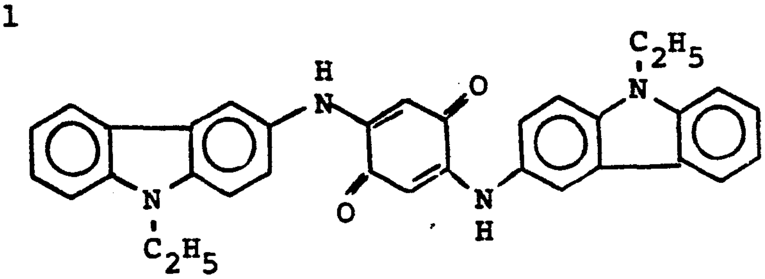

b) 20,0 g der vorgenannten Verbindung werden in 1 l Nitrobenzol auf 190°C erhitzt. 22 ml Brom werden schnell zugetropft. Man rührt 30 Min. bei 190°C nach, wobei zuletzt durch Überleiten von Luft Bromwasserstoff entfernt wird. Es wird heiß abgesaugt, mit Nitrobenzol bis zum farblosen Ablauf, dann mit Methanol gewaschen und bei 100°C getrocknet. Man erhält 31,6 g eines Dioxazins mit einem Bromgehalt von 50,3 %. Der Verbindung kommt mithin die nachstehende Formel zu

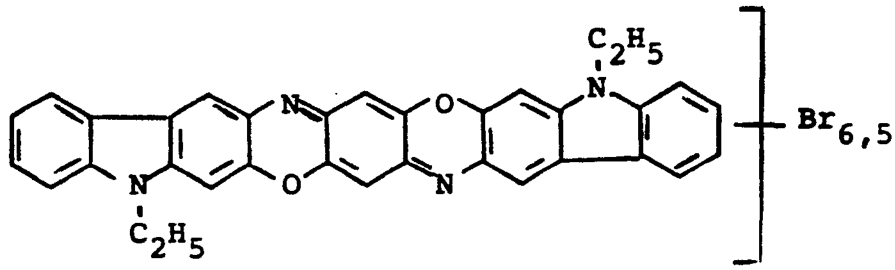

Beispiel 2

56,0 g der als Vorstufe für C.I. Pigmentviolett 23 bekannten Verbindung

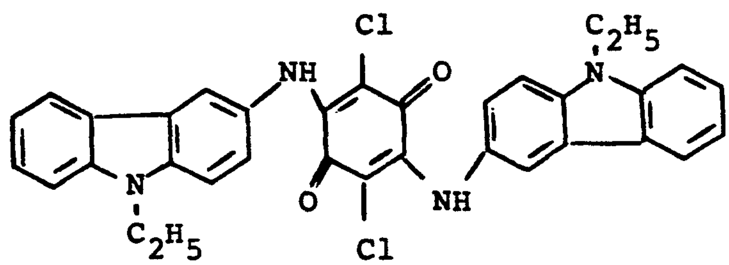

werden in 2 l siedendem Nitrobenzol mit 50 ml Brom gemäß Beispiel 1b umgesetzt und aufgearbeitet. Man erhält 82,1 g eines Dioxazins mit einem Bromgehalt von 51,6 % und einem Restgehalt an Chlor von 0,3 %.

Beispiel 3

Zu einer Mischung von 17,5 g 3-Amino-9-ethylcarbazol und 9,8 g Chloranil in 200 ml Nitrobenzol werden 6,6 g wasserfreies Natriumacetat gegeben und 1 Stunde ohne Heizung gerührt. Nach Erhitzen auf 180°C tropft man 15,5 ml Brom zu und rührt 40 Min. bei 180°C weiter,

zuletzt unter Überleiten eines Luftstroms zur Entfernung von Bromwasserstoff. Man saugt heiß ab, wäscht mit Nitrobenzol, dann mit Methanol und schließlich mit Wasser und trocknet bis 120°C. Das so erhaltene Dioxazin hat einen Bromgehalt von 52,7 % und einen Chloranteil von 2,5 %. Der Verbindung kommt somit die folgende Formel zu

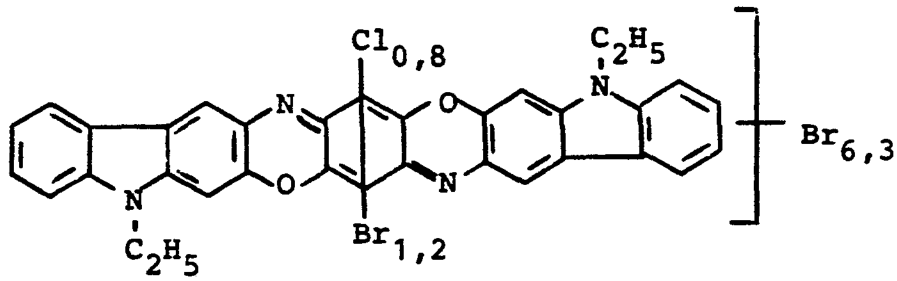

Patentansprüche:

1. Verfahren zur Herstellung von Dioxazinverbindungen der Formel

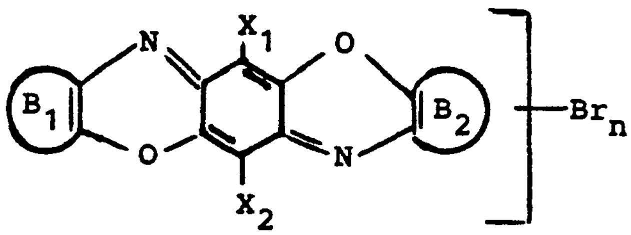

(I)

in der

$B_1$ $B_2$ ein Ringsystem mit 1, 2, 3 oder 4 carbo- und/oder heterocyclischen Ringen, die substituiert sein können und

$X_1$, $X_2$ Wasserstoff, Halogen, -R', -OR', -NHR', -NR'R", $-NH-\underset{\underset{O}{\|}}{C}R'$, $-\underset{\underset{R''}{|}}{N}-\underset{\underset{O}{\|}}{C}R'$, $-O-\underset{\underset{O}{\|}}{C}R'$, $-\underset{\underset{O}{\|}}{C}-NH_2$, $-\underset{\underset{O}{\|}}{C}-NHR'$, $-\underset{\underset{O}{\|}}{C}-NR'R''$, $-\underset{\underset{O}{\|}}{C}-OR'$

bezeichnen, wobei
R', R" für Alkyl, Cycloalkyl, Aryl, Aralkyl stehen und
n 2, 3, 4, 5, 6, 7, 8, 9, 10, 11 bedeutet, wobei die n Bromatome unabhängig voneinander sowohl H-Atome der Ringsysteme $B_1$, $B_2$ oder ihrer Substituenten als auch H-Atome von $X_1$, $X_2$ sowie $X_1$, $X_2$ selbst ersetzen können, dadurch gekennzeichnet, daß man

Verbindungen der Formel,

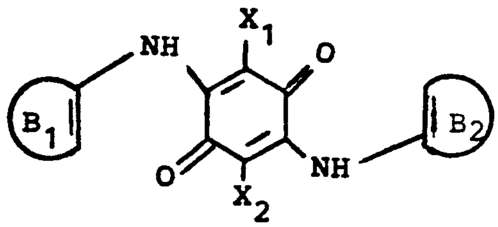

vorzugsweise in Gegenwart von Verdünnungsmitteln, mit Brom umsetzt.

2. Verfahren gemäß Anspruch 1 zur Herstellung von Verbindungen, bei denen $B_1$, $B_2$ Ringsysteme der Formeln

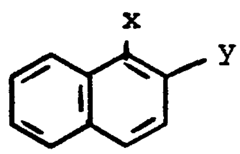

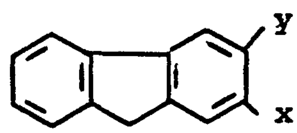

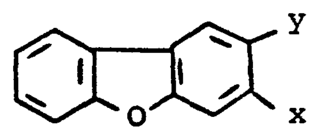

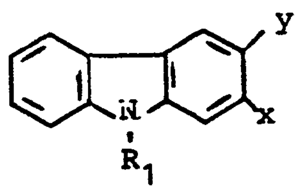

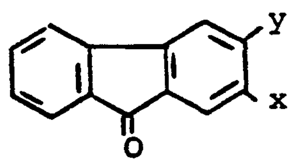

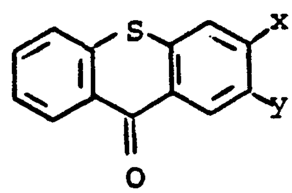

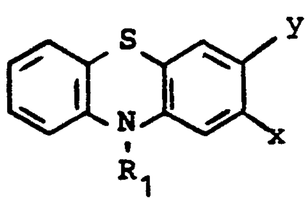

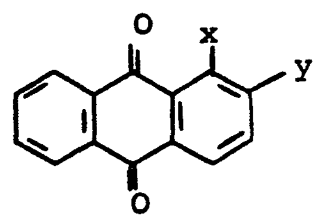

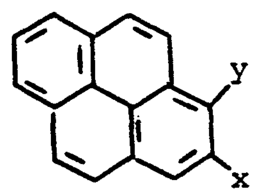

bezeichnen, bei denen $R_1$ für Wasserstoff, $R_2$-, $R_2$-O-$R_3$-, $R_2\underset{\substack{\| \\ O}}{C}-$ und $R_2-SO_2-$ steht, wobei $R_2$, $R_3$ Alkyl, Cycloalkyl, Aralkyl, Aryl bezeichnen und die mit x markierten Stellen die beim Ringschluß entstehende Bindung zum Sauerstoff und die mit y markierten Stellen die zum Stickstoff angeben.

3. Verfahren gemäß Anspruch 1 zur Herstellung von Verbindungen der Formel

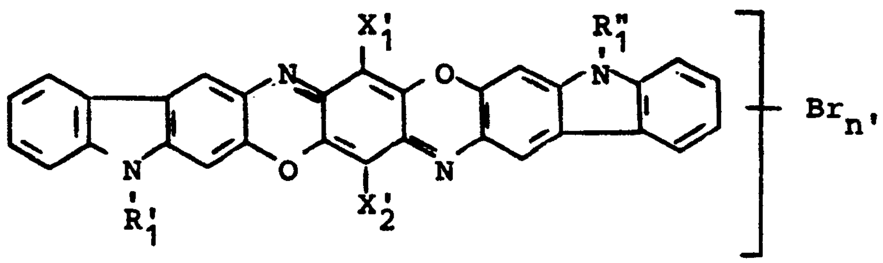

(XI)

wobei
$X_1'$, $X_2'$ die gleichen Bedeutungen wie $X_1$, $X_2$ und
$R_1'$, $R_1''$ die gleichen Bedeutungen wie $R_1$ haben und
$n'$ 4, 5, 6, 7, 8 bezeichnet.

4. Verfahren gemäß Anspruch 3 zur Herstellung von Verbindungen, bei denen $X_1' = X_2'$ und/oder $R_1' = R_1''$.

5. Verfahren gemäß den Ansprüchen 3 und 4 zur Herstellung von Verbindungen, bei denen

$X_1'$, $X_2'$ H, Cl und
$R_1'$, $R_1''$ $C_1$-$C_4$-Alkyl, vorzugsweise Ethyl sind.

6. Verfahren zur Herstellung von Verbindungen der Formel

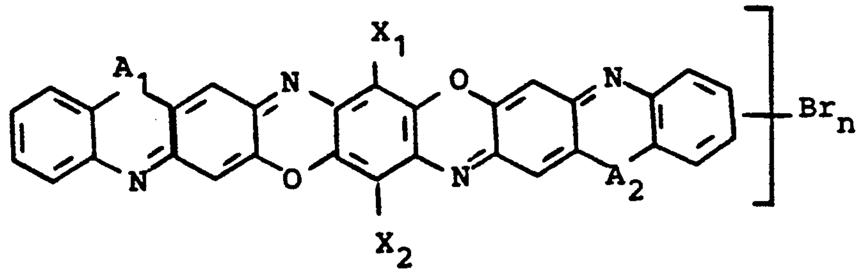

(IX)

in der
$X_1$, $X_2$ und n die in Anspruch 1 angegebenen Bedeutungen haben und
$A_1$, $A_2$ O, S oder $NR_1$, wobei $R_1$ die in Anspruch 2 angegebene Bedeutung hat, bezeichnen, dadurch gekennzeichnet, daß man Verbindungen der Formel

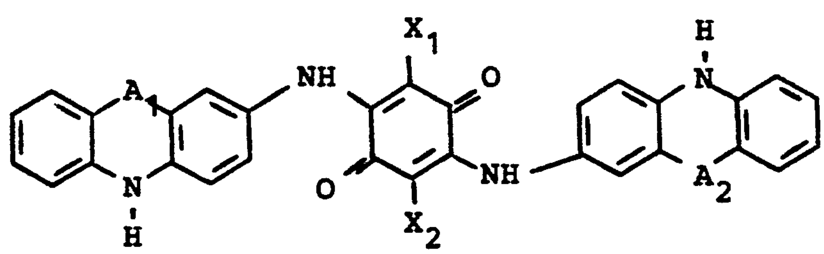

(VIII)

vorzugsweise in Gegenwart von Verdünnungsmitteln, mit Brom umsetzt.

7. Verfahren gemäß den Ansprüchen 1 - 6, dadurch gekennzeichnet, daß man in Gegenwart eines hochsiedenden, unter den Reaktionsbedingungen inerten Lösungsmittels arbeitet.

8. Verfahren gemäß den Ansprüchen 1 - 7, dadurch gekennzeichnet, daß man bei 130 - 250°C arbeitet.

9. Verfahren gemäß den Ansprüchen 1 - 7, dadurch gekennzeichnet, daß man bei 170 - 220°C arbeitet.

10. Verfahren gemäß den Ansprüchen 1 - 9, dadurch gekennzeichnet, daß man unter Druck arbeitet.